(19) Europäisches Patentamt / European Patent Office / Office européen des brevets

(11) EP 1 430 882 B1

(12) # EUROPÄISCHE PATENTSCHRIFT

(45) Veröffentlichungstag und Bekanntmachung des Hinweises auf die Patenterteilung:
**01.04.2009 Patentblatt 2009/14**

(51) Int Cl.:
***A61K 8/49*** *(2006.01)* ***A61Q 19/08*** *(2006.01)*
***A61K 31/353*** *(2006.01)*

(21) Anmeldenummer: **03028016.8**

(22) Anmeldetag: **06.12.2003**

(54) **Antioxidans-Kombinationen mit 6,7-disubstituierten 2,2-Dialkylchromanen oder -chromenen**

Combinations of antioxidants containing 6,7-disubstituted 2,2-dialkylchromanes or -chromenes

Combinaisons d'agents antioxidants contenant des 2,2-dialkylchromanes ou -chromenes disubstitués dans les positions 6 et 7

(84) Benannte Vertragsstaaten:
**AT BE BG CH CY CZ DE DK EE ES FI FR GB GR HU IE IT LI LU MC NL PT RO SE SI SK TR**

(30) Priorität: **16.12.2002 DE 10259014**

(43) Veröffentlichungstag der Anmeldung:
**23.06.2004 Patentblatt 2004/26**

(73) Patentinhaber: **Henkel AG & Co. KGaA**
**40589 Düsseldorf (DE)**

(72) Erfinder:
- **Yücel, Sevda**
  **41470 Neuss (DE)**
- **Waldmann-Laue, Marianne, Dr.**
  **40789 Monheim (DE)**

(56) Entgegenhaltungen:
**EP-A- 1 002 533** **EP-A- 1 336 403**
**WO-A-00/25731** **WO-A-01/74320**
**WO-A-01/82888** **WO-A-02/34072**
**US-A- 6 048 886**

- **MOISON R M W ET AL: "INCREASED ANTIOXIDANT POTENTIAL OF COMBINED TOPICAL VITAMIN E AND C AGAINST LIPID PEROXIDATION OF EICOSAPENTAENOIC ACID IN PIG SKIN INDUCED BY SIMULATED SOLAR RADIATION" INTERNATIONAL JOURNAL OF RADIATION BIOLOGY, TAYLOR AND FRANCIS, LONDON, GB, Bd. 78, Nr. 12, 2002, Seiten 1185-1193, XP008034680 ISSN: 0955-3002**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft topische kosmetische und pharmazeutische Zusammensetzungen, die eine synergistische Kombination eines ausgewählten 6,7-disubstituierten 2,2-Dialkylchromans oder -chromens mit mindestens einem weiteren Antioxidans, ausgewählt aus Polyphenolen, enthalten.

Bekannte 2,2-Dialkylchroman-Derivate sind vor allem Tocopherole und Tocotrienole. Die Tocopherole sind in 2-Position mit einem Methylrest und mit einem gesättigten 4',8',12'-Trimethyltridecyl-Rest substituiert und weisen in 5-, 7- und 8-Position entweder ein Wasserstoffatom oder eine Methylgruppe auf (α-, β-, γ-, δ-, $\xi_2$- und η-Tocopherole). Die Tocotrienole, auch als e-Tocopherole bezeichnet, unterscheiden sich von den vorgenannten Tocopherolen dadurch, dass in 2-Position an Stelle des gesättigten 4',8',12'-Trimethyltridecyl-Restes ein dreifach ungesättigter 4',8',12'-Trimethyltridecatrienyl-Rest aus drei Isoprenyl-Einheiten vorhanden ist. Neben ihrer Anwendung als Vitamin E wirken Tocopherole als Antioxidantien in Fetten und Ölen. Darüber hinaus besitzen Tocopherole eine Vielzahl von günstigen physiologischen Eigenschaften, z. B. Reduzierung von Muskelschäden, die auf oxidativen Streß während körperlicher Höchstleistung zurückzuführen sind, Verminderung des Risikos der Kataraktbildung, Verminderung des oxidativen Stresses bei Rauchern sowie anticarcinogene Effekte. Kosmetisch zeigen sie eine protektive Wirkung gegen Hautschäden und Hautalterung und schützen das Haar vor Witterungseinflüssen.
Neben den erwähnten Tocopherolen und Tocotrienolen sind weitere 2,2-Dialkylchroman-Derivate als kosmetische Wirkstoffe bekannt. Die Offenlegungsschrift EP 1 174 140 A1 offenbart Chromanolglycoside als Wirkstoff, der effektiv aktiven Sauerstoff und freie Radikale eliminiert. Explizit offenbart ist 2,5,7,8-Tetramethylchroman-6-ol, das in 2-Position mit einem Glucopyranosylmethyl-, Galactopyranosylmethyl-, Fructofuranosylmethyl- oder Mannopyranosylmethyl-Rest substituiert ist. Die Patentschrift US 5,811,083 offenbart das Amid aus 3,4-Dihydro-6-hydroxy-2,5,7,8-tetramethyl-1-benzopyran-2-carbonsäure (Trolox C) und Cysteamin als Antioxidans gegen Lipidperoxidation. Die Offenlegungsschrift EP 655 239 A1 offenbart die Verwendung von 2,2-Dimethylchromanen und -chromenen, die in 6- und 7-Position mit OH-, $CH_3O$- oder $CF_3CH_2O$-Gruppen substituiert sein können, als Antioxidantien in Doxorubicin-haltigen Liposomen. Daran anknüpfend, offenbart die Offenlegungsschrift EP 1 002 533 A1 die Verwendung der genannten 2,2-Dimethylchromane und -chromene als Antioxidantien in Zusammensetzungen zur topischen oder oralen Verabreichung.

Überraschend und für den Fachmann nicht vorhersehbar wurde nun gefunden, dass ausgewählte 6,7-disubstituierte 2,2-Dialkylchromane und -chromene in topischen kosmetischen und pharmazeutischen Zusammensetzungen die antioxidative Wirkung von bestimmten Antioxidantien in synergistischer Weise steigern.

Gegenstand der vorliegenden Erfindung ist eine topische kosmetische oder pharmazeutische Zusammensetzung, enthaltend mindestens ein 6,7-disubstituiertes 2,2-Dialkylchroman oder -chromen der allgemeinen Formel (I) oder (II),

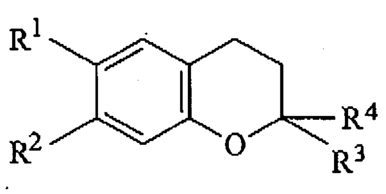

(I)

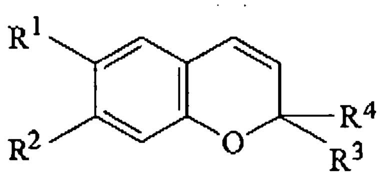

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, und mindestens ein weiteres Antioxidans, ausgewählt aus , Polyphenolen.

Die Substituenten $R^1$ und $R^2$ sind unabhängig voneinander ausgewählt aus einer OH-Gruppe, einer Methoxy-Gruppe und einer $CF_3CH_2O$-Gruppe. In einer bevorzugten Ausführungsform sind $R^1$ und $R^2$ unabhängig voneinander ausgewählt aus einer OH-Gruppe und einer Methoxy-Gruppe. In einer besonders bevorzugten Ausführungsform stellen $R^1$ eine OH-Gruppe und $R^2$ eine Methoxy-Gruppe dar.

Die Substituenten $R^3$ und $R^4$ stellen unabhängig voneinander eine $C_1$-$C_4$-Alkylgruppe dar. Unter $C_1$-$C_4$-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind $R^3$ und $R^4$ unabhängig voneinander ausgewählt aus einer Methyl-, Ethyl-, n-Propyl-, Isopropyl- und einer n-Butyl-Gruppe. Besonders bevorzugt stellen $R^3$ und $R^4$ unabhängig voneinander eine Methyl- oder Ethylgruppe dar. Außerordentlich bevorzugt ist, dass $R^3$ und $R^4$ identisch sind.

Erfindungsgemäß bevorzugt sind die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) werden erfindungsgemäß in Mengen von 0,001 - 5 Gew.-%, bevorzugt 0,005 - 2 Gew.-% und besonders bevorzugt 0,01 - 1 Gew.-%, jeweils bezogen auf die gesamte Zusammensetzung, eingesetzt.

Die Antioxidantien, deren antioxidative Wirkung in der erfindungsgemäßen Kombination mit den 6,7-disubstituierten 2,2-Dialkylchromanen oder -chromenen der Formel (I) oder (II) gesteigert wird, werden sowohl zur Stabilisierung der Fette gegen Autoxidation als auch als Radikalfänger sowie zur nicht-therapeutischen Hautpflege und/oder zur nicht-therapeutischen vorbeugenden Behandlung der Hautalterung verwendet.

Die erfindungsgemäß in ihrer Wirkung gesteigerten Antioxidantien sind aus Polyphenolen ausgewählt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Pinienrinde und Eichenrinde sowie anderer Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda.

Erfindungsgemäß werden die Polyphenole in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt.

Die Gesamtmenge der zusätzlichen Antioxidantien, das heißt ohne die 6,7-disubstituierten 2,2-Dialkylchromane oder -chromene, beträgt 0,001 - 10 Gew.-%, vorzugsweise 0,05 - 5 Gew.-% und insbesondere 0,1 - 2 Gew.-%, bezogen auf das Gesamtgewicht der erfindungsgemäßen Zusammensetzung.

Die erfindungsgemäß hergestellten topischen kosmetischen oder pharmazeutischen Zusammensetzungen können sowohl als Leave-on-Produkt als auch als Rinse-off-Produkt formuliert werden. Geeignete Leave-on-Produkte sind Cremes, Salben, Pflegestifte, Make-ups, Lotionen, Masken, Packungen und Pflasterbeschichtungen, Sprays, Moussen sowie Deodorants und Antitranspirantien insbesondere zur Unterarmbehandlung, die in Form von O/W-Emulsionen, W/O-Emulsionen, O/W/O-Emulsionen, W/O/W-Emulsionen, O/W-Mikroemulsionen, W/O-Mikroemulsionen, wässrigen, alkoholischen oder wässrigalkoholischen Gelen sowie als Öl vorliegen können. Geeignete Rinse-off-Produkte sind Waschgele, Waschcremes, Duschgele, Duschbäder, Seifen, Shampoos, Haarkonditioniermittel, Haarfärbezusammensetzungen, Haarwell- und Haarglättungsmittel sowie Tränkemulsionen und -lösungen für feste Substrate zur Reinigung und/oder Pflege der Haut und/oder der Haare.

Die erfindungsgemäß verwendeten 6,7-disubstituierten 2,2-Dialkylchromane oder - chromene liegen als pulverförmiger Rohstoff vor und können unterhalb von ca. 40°C einfach in die vorgefertigten kosmetischen oder pharmazeutischen Zusammensetzungen eingearbeitet werden. Das vorherige Lösen der Chroman- oder Chromenderivate in einem geeigneten Lösemittel erhöht ihre Dispersibilität in der Formulierung. Geeignete Lösemittel sind die einwertigen $C_2$ - $C_4$-Alkohole, speziell Ethanol, Propanol, Isopropanol und Butanol, weiterhin Propylenglycol sowie Polyethylenglycole, speziell PEG-4, PEG-200 und PEG-400. Weiterhin geeignet sind polare Ölkomponenten, insbesondere die Triglyceride von gesättigten und ungesättigten $C_8$ - $C_{18}$-Fettsäuren. Bevorzugte Beispiele sind natürliche Triglyceride, z. B. Weizenkeimöl, sowie synthetische Triglyceride, z. B. das Handelsprodukt Myritol[R] 318. Schließlich lassen sich die erfindungsgemäß verwendeten Chroman- oder Chromen-Derivate mit Hilfe geeigneter Lösungsvermittler dispergieren. Geeignet sind insbesondere ethoxylierte Sorbitanfettsäuremonoester von $C_{12}$ - $C_{18}$-Fettsäuren, bevorzugt insbesondere Polysorbate-20 und Polysorbate-80.

Die erfindungsgemäßen Zusammensetzungen können weiterhin mindestens ein Polyol als Feuchthaltemittel enthalten. Bevorzugte Polyole sind Diole, Triole und höhere Alkohole, Polyglycerine, Polyethylenglycole sowie Mono- und Disaccharide. Unter den Diolen eignen sich $C_2$-$C_{12}$-Diole, insbesondere 1,2-Propylenglycol, Butylenglycole wie z. B. 1,2-Butylenglycol, 1,3-Butylenglycol und 1,4-Butylenglycol, Hexandiole, wie z. B. 1,6-Hexandiol. Weiterhin bevorzugt geeignet sind Glycerin und Polyglycerine, insbesondere Diglycerin und Triglycerin, 1,2,6-Hexantriol, sowie die Polyethylenglycole (PEG) PEG-400, PEG-600, PEG-1000, PEG-1550, PEG-3000 und PEG-4000.
Erfindungsgemäß geeignete Monosaccharide sind z. B. Glucose, Fructose, Galactose, Arabinose, Ribose und Mannose sowie Aminozucker wie z. B. Glucosamin oder Galactosamin.
Erfindungsgemäß geeignete Disaccharide sind aus zwei Monosaccharideinheiten zusammengesetzt, z. B. Saccharose oder Lactose. Ein besonders bevorzugtes Disaccharid ist Saccharose. Ebenfalls besonders bevorzugt ist die Verwendung von Honig, der überwiegend Glucose und Saccharose enthält.

Desweiteren lassen sich die erfindungsgemäß verwendeten Zusammensetzungen mit weiteren kosmetischen Wirkstoffen, z. B. Proteinhydrolysaten oder Pflanzenextrakten, formulieren. Viele dieser Substanzen zeigen eine Antifalten- oder Anti-Age-Wirkung, so dass entsprechende erfindungsgemäß verwendeten Zusammensetzungen auch als Antifalten- oder Anti-Age-Mittel verwendet werden können.

Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Kartoffel-, Mandel-, Erbsen- und Reisproteinhydrolysate. Entsprechende Handelsprodukte sind z. B. DiaMin® (Diamalt), Gluadin® (Cognis), Lexein® (Inolex) und Crotein® (Croda).
Wenngleich der Einsatz der Proteinhydrolysate als solche bevorzugt ist, können an ihrer Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon® (Cognis), Gluadin® (Cognis), Lexein® (Inolex), Crolastin® oder Crotein® (Croda).
Erfindungsgemäß einsetzbar sind auch kationisierte Proteinhydrolysate, wobei das zugrunde liegende Proteinhydrolysat vom Tier, von der Pflanze, von marinen Lebensformen oder von biotechnologisch gewonnenen Proteinhydrolysaten stammen kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate seien aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen.
Erfindungsgemäß sind die Proteinhydrolysate und deren Derivate in Mengen von 0,01 - 10 Gew.-% bezogen auf die gesamte Zusammensetzung, enthalten. Mengen von 0,1 bis 5 Gew.%, insbesondere 0,1 bis 3 Gew.-%, sind besonders bevorzugt.

Die erfindungsgemäß hergestellten Zusammensetzungen können weiterhin Ölkomponenten enthalten. Geeignete Ölkomponenten sind ausgewählt aus den Estern von gesättigten und ungesättigten, linearen und verzweigten $C_3$-$C_{22}$-Fettsäuren und den Dimeren ungesättigter $C_{12}$-$C_{22}$-Fettsäuren (Dimerfettsäuren) mit a) einwertigen linearen, verzweigten und cyclischen $C_2$-$C_{18}$-Alkanolen, b) mehrwertigen linearen und verzweigten $C_2$-$C_6$-Alkanolen, c) Polyglycerinen der Formel $CH_2OH-CHOH-CH_2[-O-CH_2-CHOH-CH_2]_n-O-CH_2-CHOH-CH_2OH$ mit n = 0 - 8, und d) den Dicarbonsäureestern von linearen und verzweigten $C_2$-$C_{10}$-Alkanolen, den Benzoesäureestern von linearen und verzweigten $C_{10}$-$C_{20}$-Alkanolen, den $C_{12}$-$C_{22}$-Alkanolestern ein- und mehrwertiger $C_2$-$C_7$-Hydroxycarbonsäuren, die aromatisch sein können, sowie Mischungen dieser Komponenten.
Beispiele für erfindungsgemäß besonders geeignete Fettsäureester einwertiger linearer oder verzweigter $C_2$-$C_{18}$-Alkanole (Typ a) sind Octylethylhexanoat, Isopropylpalmitat, Hexyllaurat, Isopropylmyristat, ein Gemisch der Ester von Caprylsäure und Caprinsäure mit $C_{12}$-$C_{18}$-Fettalkoholen, das als Handelsprodukt Cetiol® LC (Cognis) erhältlich ist, $C_{12}$-$C_{15}$-Alkyloctanoat, Isodecylneopentanoat, Isononylisononanoat, 2-Ethylhexylpalmitat (z. B. Cegesoft® C 24 von Cognis), 2-Ethylhexylstearat, Cetearylisononanoat (Mischung aus Cetylisononanoat und Stearylisononanoat, z. B. Cetiol® SN (Cognis)), Cetearyloctanoat, Ethylstearat, Isopropylstearat, Butylstearat und Myristylpropionat. Weiterhin geeignet sind die Monoester des Isosorbid (1,4:3,6-Dianhydro-D-glucit) mit $C_2$-$C_{24}$-Fettsäuren, z. B. das Isosorbidmonolaurat.
Beispiele für erfindungsgemäß besonders geeignete $C_8$-$C_{22}$-Fettsäureester mehrwertiger $C_2$-$C_6$-Alkanole (Typ b) sind als pflanzliche und tierische Öle vorkommende Fettsäuretriglycerid-Öle, z. B. Sonnenblumenöl, Sojaöl, Sesamöl, Haselnußöl, Mandelöl, Olivenöl, sowie Distelöl (Safloröl). Besonders bevorzugt ist Distelöl. Erfindungsgemäß außerordentlich gut geeignet sind weiterhin synthetische Fettsäuretriglycerid-Öle. Besonders bevorzugt sind die Triglyceride von gesättigten $C_8$-$C_{10}$-Fettsäuren, wobei die Fettsäuren sowohl unverzweigt, wie z. B. bei den Handelsprodukten Myritol® 318 und Myritol® 331 (Cognis) oder Miglyol® 812 (Hüls), als auch verzweigt sein können, wie z. B. bei den Handelsprodukten Estol® GTEH 3609 (Uniqema) oder Myritol® GTEH (Cognis).
Weitere geeignete Ester dieses Typs sind die Fettsäureester des 1,2-Propylenglycols, des Neopentylglycols, z. B. Neopentylglycoldiheptanoat, des Trimethylolpropans und des Pentaerythrits, wobei die 1,2-Propylenglycolester bevorzugt sind. Beispiele für erfindungsgemäß bevorzugte Ölkomponenten sind die 1,2-Propylenglycoldiester von gesättigten,

unverzweigten $C_8$-$C_{10}$-Fettsäuren, besonders bevorzugt die Handelsprodukte Myritol® PC und Miglyol® 840.
Beispiele für erfindungsgemäß besonders geeignete Fettsäureester von Polyglycerinen (Typ c) sind Polyglyceryl-2-dipolyhydroxystearat (Handelsprodukt Dehymuls® PGPH von Cognis), Polyglyceryl-3-diisostearat (Handelsprodukt Lameform® TGI von Cognis) und der Diester der Dilinolsäure mit Polyglyceryl-3-diisostearat (Handelsprodukt Isolan® PDI von Th. Goldschmidt).
Beispiele für erfindungsgemäß besonders geeignete Dicarbonsäureester (Typ d) sind Diisopropylsebacat, Diisopropyladipat, Dioctylmalat, Dioctylmaleat, Dicaprylylmaleat, Dioctyladipat, Di-n-butyladipat, Diisooctylsuccinat, Di-(2-hexyldecyl)-succinat und Diisotridecylacelaat.
Beispiele für erfindungsgemäß besonders geeignete Benzoesäureester von linearen oder verzweigten $C_{10}$-$C_{20}$-Alkanolen sind unter dem Warenzeichen Finsolv® (Hersteller: Finetex) erhältlich. Besonders bevorzugt sind die Handelsprodukte Finsolv® TN (Benzoesäure-C12-C15-alkylester), Finsolv® SB (Benzoesäureisostearylester) und Finsolv® BOD (Benzoesäureoctyldocecylester).
Als $C_8$-$C_{22}$-Fettalkoholester einwertiger oder mehrwertiger $C_2$-$C_7$-Hydroxycarbonsäuren sind die Ester der Glycolsäure, Milchsäure, Äpfelsäure, Weinsäure, Citronensäure und Salicylsäure erfindungsgemäß besonders geeignet. Solche Ester auf Basis von linearen $C_{14/15}$-Alkanolen und von in 2-Position verzweigten $C_{12/13}$-Alkanolen sind unter dem Warenzeichen Cosmacol® von der Fa. Nordmann, Rassmann GmbH & Co, Hamburg, zu beziehen. Besonders bevorzugt sind die Handelsprodukte Cosmacol® ESI, Cosmacol® EMI und Cosmacol® ETI.
Die Ölkomponenten sind in einer Menge von 1 - 20 Gew.-% in der erfindungsgemäßen Zusammensetzung enthalten. Bevorzugt ist ein Gehalt von 3 - 15 Gew.-%, besonders bevorzugt von 4 - 10 Gew.-% an Ölkomponenten, bezogen auf die Gesamtzusammensetzung.

Die erfindungsgemäß verwendeten Zusammensetzungen können weiterhin nichtionische Coemulgatoren enthalten. Bevorzugte Coemulgatoren sind lineare $C_{12}$-$C_{22}$-Fettalkohole und $C_{12}$-$C_{22}$-Fettsäurepartialester von $C_2$-$C_6$-Polyolen.
Geeignete Fettalkohole sind z.B. Cetylalkohol, Stearylalkohol, Oleylalkohol, Erucaalkohol, Ricinolalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.
Bevorzugte Polyol-Fettsäurepartialester sind Mono- und -diester von Glycerin oder 1,2-Propylenglycol mit gesättigten und ungesättigten linearen und verzweigten $C_{12}$-$C_{22}$-Fettsäuren sowie die Mono-, Sesqui- und -triester von Sorbitan mit gesättigten und ungesättigten linearen und verzweigten $C_{18}$-$C_{22}$-Fettsäuren. Besonders bevorzugt sind Glycerinmonostearat, Glycerinmonopalmitat, Glycerinmonolaurat, Glycerinmonooleat, Glycerindistearat, Glycerindipalmitat, Glycerindilaurat, Glycerindioleat sowie technische Gemische dieser Komponenten, weiterhin Sorbitanmonostearat, Sorbitanmonooleat, Sorbitansesquioleat, Sorbitantristearat, Sorbitantrioleat sowie technische Gemische dieser Komponenten. Die Polyol-Fettsäurepartialester können ethoxyliert sein.

Weiterhin können außer den vorgenannten polaren Ölkomponenten und Coemulgatoren auch unpolare Ölkomponenten sowie Fettstoffe, die bei Raumtemperatur fest sind, enthalten sein. Zu diesen übrigen Öl- und Fettkomponenten zählen Fettsäuren sowie natürliche und synthetische Wachse, die sowohl in fester Form als auch flüssig oder in wäßriger Dispersion vorliegen können, sowie natürliche und synthetische unpolare kosmetische Öle.
Als Fettsäuren können eingesetzt werden lineare und/oder verzweigte, gesättigte und/oder ungesättigte $C_{8-30}$-Fettsäuren. Bevorzugt sind $C_{10-22}$-Fettsäuren. Beispiele sind die Isostearinsäuren und Isopalmitinsäuren. Weitere geeignete Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen.
Als natürliche oder synthetische Wachse können erfindungsgemäß eingesetzt werden feste Paraffine oder Isoparaffine, Pflanzenwachse wie Candelillawachs, Carnaubawachs, Espartograswachs, Fruchtwachse und Sonnenblumenwachs, Bienenwachse und andere Insektenwachse, Ozokerite, Ceresin, Walrat sowie Microwachse aus Polyethylen oder Polypropylen. Weiterhin geeignet sind die Triglyceride gesättigter und gegebenenfalls hydroxylierter $C_{16-30}$-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 — 6 C-Atomen, Fettsäuremonoalkanolamide mit einem $C_{12-22}$-Acylrest und einem $C_{2-4}$-Alkanolrest, synthetische Fettsäure-Fettalkoholester, z. B. Stearylstearat oder Cetylpalmitat, Esterwachse aus natürlichen Fettsäuren und synthetischen $C_{20-40}$-Fettalkoholen (INCI-Bezeichnung C20-40 Alkyl Stearate) und Vollester aus Fettalkoholen und Di- und Tricarbonsäuren, z. B. Dicetylsuccinat oder Dicetyl-/stearyladipat, sowie Mischungen dieser Substanzen.
Vorteilhafte kosmetische Ölkörper, die erfindungsgemäß eingesetzt werden können, sind beispielsweise flüssige Paraffinöle, Isoparaffinöle und synthetische Kohlenwasserstoffe wie 1,3-Di-(2-ethyl-hexyl)-cyclohexan (Cetiol® S) sowie Di-n-alkylether mit insgesamt 12 bis 36, insbesondere 12 bis 24 C-Atomen, wie Di-n-octylether, Di-n-decylether, Di-nnonylether, Di-n-undecylether, n-Hexyl-n-octylether und n-Octyl-n-decylether. 1,3-Di-(2-ethyl-hexyl)-cyclohexan (Cetiol® S) und Di-n-octylether (Cetiol® OE) können bevorzugt sein. Weiterhin können symmetrische, unsymmetrische

oder cyclische Ester der Kohlensäure mit Fettalkoholen, beispielsweise beschrieben in der DE-OS 197 56 454, Glycerincarbonat oder Dicaprylylcarbonat (Cetiol® CC) eingesetzt werden.
Weitere erfindungsgemäß vorteilhaft einzusetzende Fettstoffe sind Siloxane. Die Siloxane können als Öle, Harze oder Gums vorliegen. Bevorzugte Siloxane sind Polydialkylsiloxane, wie z. B. Polydimethylsiloxan, Polyalkylarylsiloxane, wie z. B. Polyphenylmethylsiloxan, Polydialkylsiloxane, die Amin- und/oder Hydroxy-Gruppen enthalten sowie cyclische Silicone (INCI-Bezeichnung: Cyclomethicone), bevorzugt Octamethylcyclotetrasiloxan, Decamethylcyclopentasiloxan und Dodecamethylcyclohexasiloxan.

Weiterhin können die erfindungsgemäß verwendeten Zusammensetzungen mindestens ein Polymer enthalten. Die Polymere sind ausgewählt aus natürlichen und synthetischen anionischen, kationischen, nichtionischen und amphoteren Polymeren. Erfindungsgemäß bevorzugt sind synthetische und natürliche anionische und nichtionische Polymere.
Geeignete anionische Polymere enthalten Carboxylat- und/oder Sulfonatgruppen und als Monomere beispielsweise Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.
Innerhalb dieser Ausführungsform kann es bevorzugt sein, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionische Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 - 55 Mol-% Acrylamid und 30 - 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel® 305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel® EG als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise die Handelsprodukte Carbopol®. Weitere bevorzugte anionische Copolymere sind solche, die als Monomere 80 - 98 Gew.-% gewünschtenfalls substituierte Acrylsäure und 2 - 20 Gew.-% $C_{12}$-$C_{30}$-Fettalkoholmethacrylsäureester enthalten und vernetzt sein können. Solche Verbindungen sind z. B. die Handelsprodukte Pemulen®.
Unter amphoteren Polymeren versteht man sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen bzw. -COOHoder $SO_3H$-Gruppen enthalten. Ein Beispiel für ein erfindungsgemäß einsetzbares amphoteres Polymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.
Die erfindungsgemäßen Mittel können weiterhin synthetische nichtionische Polymere enthalten. Geeignete nichtionische synthetische Polymere sind Polyvinylpyrrolidone und Vinylpyrrolidon/Vinylester-Copolymere, z. B. die Handelsprodukte Luviskol® (BASF), sowie Polyvinylalkohole.
Bei kationischen Polymeren unterscheidet man "temporär" und "permanent" kationische Polymere. Temporär kationische Polymere enthalten üblicherweise eine Aminogruppe, die bei bestimmten pH-Werten als quartäre Ammoniumgruppe vorliegt. Bevorzugt sind Chitosan und dessen Derivate, z. B. die Produkte Hydagen® CMF, Hydagen® HCMF, Kytamer® PC und Chitolam® NB/101. Besonders gut geeignete Chitosane weisen einen Deacetylierungsgrad von wenigstens 80 % und ein Molekulargewicht von $5 \cdot 10^5$ bis $5 \cdot 10^6$ g/mol auf. Zur Herstellung erfindungsgemäßer Zubereitungen muß das Chitosan in die Salzform überführt werden. Hierzu geeignete Säuren sind z. B. Essigsäure, Glycolsäure, Weinsäure, Apfelsäure, Citronensäure, Milchsäure, 2-Pyrrolidon-5-carbonsäure, Benzoesäure und Salicylsäure.

Weitere verwendbare kationische Polymere sind beispielsweise quaternisierte CelluloseDerivate, z. B. die Handelsprodukte Celquat® und Polymer JR®, insbesondere Celquat® H 100, Celquat® L 200 und Polymer JR®400, kationische Alkylpolyglycoside gemäß der DE-PS 44 13 686, kationisierter Honig, kationische Guar-Derivate, insbesondere die Produkte Cosmedia®Guar und Jaguar®, Polysiloxane mit quaternären Gruppen, wie z.B. die Handelsprodukte Q2-7224 (Dow Corning), Dow Corning® 929 Emulsion, SM-2059 (General Electric), SLM-55067 (Wacker) sowie Abil®-Quat 3270 und 3272 (Th. Goldschmidt), sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette. Gleichfalls als kationische Polymere eingesetzt werden können die unter den Bezeichnungen Polyquaternium-24 bekannten Polymere.
Ebenfalls als kationisches Polymer verwendbar ist das, gewünschtenfalls vernetzte, Poly(methacryloyloxyethyltrimethylammoniumchlorid) mit der INCI-Bezeichnung Polyquaternium-37. Polyquaternium-37 wird häufig in Form einer nichtwässrigen Polymerdispersion eingesetzt. Solche Polymerdispersionen sind unter den Bezeichnungen Salcare® SC 95 und Salcare® SC 96 im Handel erhältlich.
Copolymere aus Methacryloyloxyethyltrimethylammoniumchlorid und nichtionischen Monomeren, bevorzugt Acrylamid, Methacrylamid, Acrylsäure-$C_{1-4}$-alkylester und Methacrylsäure-$C_{1-4}$-alkylester, die gegebenenfalls vernetzt sein können, sind im Handel unter dem Namen Salcare® SC 92 erhältlich.
Es ist erfindungsgemäß auch möglich, dass die verwendeten Zubereitungen mehrere, insbesondere zwei verschiedene Polymere gleicher Ladung und/oder jeweils ein ionisches und ein amphoteres und/oder ein synthetisches nichtionisches Polymer enthalten.

Als weitere Hilfsmittel können die erfindungsgemäßen Zusammensetzungen übliche galenische Hilfsstoffe in den gebräuchlichen Mengen enthalten. Solche Hilfsstoffe sind z.B. Verdickungsmittel für die wässrige Phase, z.B. vom Typ der Schichtsilikate oder der pyrogenen Kieselsäuren. Weitere Hilfsstoffe sind Konservierungsstoffe wie z. B. Phenoxyethanol, p-Hydroxybezoesäureester, Sorbinsäure, Komplexbildner (z.B. Trilon B) sowie Farb- und Duftstoffe.

Weiterhin können Emulgatoren enthalten sein. Bevorzugte Emulgatoren sind ausgewählt aus der Gruppe der nichtionischen Ethylenoxid-Addukte, oder einer Kombination aus einem hydrophilen, nichtionischen Emulgator mit einem HLB-Wert von bevorzugt 10-19 und einem lipophilen Coemulgator. Unter dem HLB-Wert soll dabei eine Größe verstanden werden, die aus der Struktur des Emulgators errechnet werden kann gemäß

$$HLB = \frac{100 - L}{5}$$

worin L der Gewichtsanteil (in %) der lipophilen Gruppen, z. B. der Fettalkyl- bzw. Fettacylgruppen im Emulgator ist.

Erfindungsgemäß verwendbare hydrophile nichtionische Emulgatoren sind beispielsweise

- Anlagerungsprodukte von 4 bis 30 Mol Ethylenoxid und/oder 0 bis 5 Mol Propylenoxid an lineare $C_8$-$C_{30}$-Fettalkohole, an $C_{12}$-$C_{22}$-Fettsäuren und an $C_8$-$C_{15}$-Alkylphenole,
- $C_{12}$-$C_{22}$-Fettsäuremono- und -diester von Anlagerungsprodukten von 1 bis 30 Mol Ethylenoxid an $C_3$-$C_6$-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten $C_8$-$C_{22}$-Fettsäuren,
- Sterole (Sterine),
- Phospholipide,
- Fettsäureester von Zuckern und Zuckeralkoholen, wie Sorbit,
- Polyglycerine und Polyglycerinderivate, beispielsweise Polyglycerinpoly-12-hydroxystearat (Handelsprodukt Dehymuls® PGPH).

Bevorzugte hydrophile Emulgatoren sind die Ethylenoxid-Anlagerungsprodukte an lineare $C_{16}$-$C_{30}$-Fettalkohole oder an Partialester von $C_3$-$C_6$-Polyolen mit $C_{12}$-$C_{22}$-Fettsäuren. Es kann besonders bevorzugt sein, als hydrophile Emulgatoren Alkylpolyglycoside der Formel RO - $(Z)_x$ einzusetzen, in der R eine $C_8$-$C_{22}$-Alkyl- oder Alkenylgruppe, Z einen Monosaccharidrest, insbesondere Glucose, und dessen Oligomerisationsgrad x eine Zahl von 1,1 bis 5, insbesondere von 1,2 bis 1,4 darstellt.
Die Emulgatoren sind in einer Menge von 0,2 - 10 Gew.-%, bevorzugt 0,5 - 8 Gew.-% und besonders bevorzugt 1 - 7 Gew.-%, jeweils bezogen auf die gesamte Zusammensetzung, enthalten.

Ein weiterer Gegenstand der Erfindung ist die nicht-therapeutische Verwendung mindestens eines 6,7-disubstituierten 2,2-Dialkylchromans oder -chromens der allgemeinen Formel (I) oder (II),

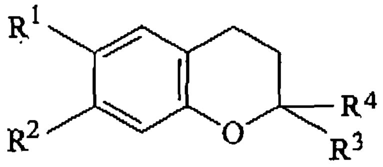

(I)

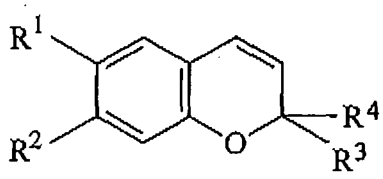

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, in topischen kosmetischen und pharmazeutischen Zusammensetzungen zur synergistischen Steigerung der antioxidativen Wirkung von Antioxidantien, ausgewählt aus , Polyphenolen.

Die folgenden Beispiele sollen den Gegenstand der Erfindung näher erläutern, ohne ihn hierauf zu beschränken.

**Beispiele**

**Erfindungsgemäße Beispielrezepturen (Mengenangaben in Gewichtsteilen)**

| | | 2 | 3 |
|---|---|---|---|
| Phase 1 | Distelöl | 3,0 | 3,0 |
| | Myritol® PC | 3,5 | 3,5 |
| | Lanette® 22 | 3,0 | 3,0 |
| | Cutina® GMS-V | 3,0 | 3,0 |
| | Stenol® 16/18 | 2,0 | 2,0 |
| | Isopropylstearat | 6,0 | 6,0 |
| | Baysilon® M350 | 1,0 | 1,0 |
| | Uvinul® T 150 | 2,5 | - |
| | Propylparaben | 0,2 | 0,2 |
| Phase 2 | Glycerin | 5,0 | 5,0 |
| | Methylparaben | 0,2 | 0,2 |
| | Wasser | 40,0 | 50,0 |
| Phase 3 | Citronensäure | 0,1 | 0,1 |
| Phase 4 | Lipochroman-6 | 0,01 | 0,01 |
| | Natriumascorbylphosphat | - | - |
| | Sepivinol R | 0,5 | - |
| | Crodarom Chardonnay | - | 0,05 |
| | Coenzym Q 10 | - | - |
| | Tinogard TT | - | - |
| | Isoascorbinsäure | - | - |
| | Rosmarinsäure | - | - |
| | Lycopin | - | - |

(fortgesetzt)

| | | 2 | 3 |
|---|---|---|---|
| Phase 5 | Sepigel® 305 | 2,0 | 2,0 |
| | Wasser | ad 100 | ad 100 |

Herstellung:

Fett- und Wasserphase (Phase 1 und Phase 2) wurden getrennt voneinander auf etwa 80° C erhitzt. Die Wasserphase wurde unter dem Homogenisator vorgelegt. Dann wurde Phase 1 hinzugegeben und erneut etwa 1 Minute lang homogenisiert. Nach dem Abkühlen auf etwa 30° C wurden Lipochroman-6 und das zweite Antioxidans zugegeben und der pH-Wert mit Citronensäure auf einen Wert im Bereich von 4,8 - 5,2 eingestellt. Anschließend wurde das Polymer (Sepigel® 305) hinzugegeben. Es bildete sich eine stabile Emulsion, nach dem Abkühlen auf 20° C eine glatte Creme von hoher Stabilität.

| Komponente | 10 |
|---|---|
| Hostaphat KL 340 N | 1,5 |
| Ethylhexylpalmitat | 3,0 |
| Paraffinöl | 8,0 |
| Tocopherylacetat | 0,01 |
| Lipochroman-6® | 0,01 |
| Carbomer | 0,2 |
| NaOH | ad pH 4,8 - 5,2 |
| Propylenglycol | 10,0 |
| Methylparaben | 0,5 |
| Propylparaben | 0,5 |
| ß-Carotin | - |
| Ascorbinsäure | 0,05 |
| 2,6-Di-tert.-butyl-4-methylphenol | - |
| Crodarom Chardonnay | 0,5 |
| Vegeles Phytofiltre AD | - |
| Wasser | ad 100 |

Herstellung:

Fett- und Wasserphase wurden getrennt voneinander auf etwa 80° C erhitzt. Die Wasserphase wurde unter dem Homogenisator vorgelegt. Dann wurde die Fettphase hinzugegeben und etwa 1 Minute lang homogenisiert. Nach dem Abkühlen auf etwa 30° C wurden Lipochroman-6, das weitere erfindungsgemäße Antioxidans und die übrigen kosmetischen Wirkstoffe zugegeben und der pH-Wert mit Citronensäure auf einen Wert

im Bereich von 4,8 - 5,2 eingestellt. Anschließend wurde das Carbomer hinzugegeben.

| **Liste der verwendeten Inhaltsstoffe** | | | |
|---|---|---|---|
| **Komponente** | **INCI-Bezeichung** | | **Hersteller** |
| Baysilonöl® M 350 | Dimethicone | Polydimethylsiloxan | GE-Bayer-Silicones |
| Controx® KS | Tocopherol, Hydrogenated Palm Glycerides Citrate | Tocopherol-Mono-diglyceridecitrat-Gemisch | Cognis |

(fortgesetzt)

| Liste der verwendeten Inhaltsstoffe | | | |
|---|---|---|---|
| **Komponente** | **INCI-Bezeichung** | | **Hersteller** |
| Crodarom Chardonnay | Vitis Vinifera (Grape) Seed Extract | | Croda |
| Cutina® GMS-V | Glyceryl Stearate | Glycerin-mono-di-palmitatstearat, pflanzlich | Cognis |
| Kessco® IPS | Isopropyl Stearate | | Akzo Nobel |
| Lanette® 22 | Behenyl Alcohol | | Cognis |
| Lipochroman-6 | Dimethylmethoxy Chromanol | 4-Dihydro-7-methoxy-2,2-dimethyl-2H-1-benzopyran-6-ol | Lipotec S.A: |
| Myritol® PC | Propylene Glycol Dicaprylate/ Dicaprate | | Cognis |
| Sepigel® 305 | Polyacrylamide, C13-14 Isoparaffin, Laureth-7 | | SEPPIC |
| Sepivinol R | Wine Extract | Polyphenolreicher Extrakt aus Rotwein | SEPPIC |
| Stenol® 16/18 | Cetearyl Alcohol | Cetyl-/Stearylalkohol (1 : 2) | Cognis |
| Tinogard TT | Tetradibutyl Pentaerithrityl Hydroxycinnamate | | Ciba |
| Uvinul® T 150 | Octyl Triazone | | BASF |
| Vegeles Phytofiltre AD | Water and Propylene Glycol and Bioflavonoids and Hydrolyzed Adansonia Digitata Extract and Dextrin | | Cognis |

## Patentansprüche

**1.** Topische kosmetische oder pharmazeutische Zusammensetzung, enthaltend mindestens ein 6,7-disubstituiertes 2,2-Dialkylchroman oder -chromen der allgemeinen Formel (I) oder (II),

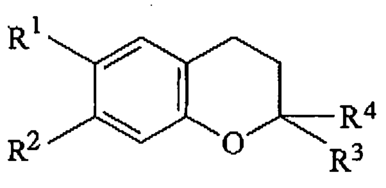

(I)

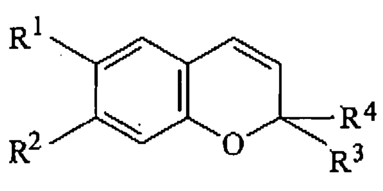

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, und mindestens ein weiteres Antioxidans, ausgewählt aus Polyphenolen.

**2.** Zusammensetzung gemäß Anspruch 1, **dadurch gekennzeichnet, dass** $R^1$ und $R^2$ unabhängig voneinander eine

OH-Gruppe oder eine Methoxy-Gruppe darstellen.

3. Zusammensetzung gemäß einem der Ansprüche 1 oder 2, **dadurch gekennzeichnet, dass** $R^3$ und $R^4$ unabhängig voneinander ausgewählt sind aus einer Methyl-, Ethyl-, n-Propyl-, Isopropyl- und n-Butylgruppe.

4. Zusammensetzung gemäß einem der vorstehenden Ansprüche, **dadurch gekennzeichnet, dass** $R^1$ eine OH-Gruppe, $R^2$ eine Methoxy-Gruppe und $R^3$ und $R^4$ eine Methylgruppe darstellen.

5. Zusammensetzung gemäß einem der vorstehenden Ansprüche, **dadurch gekennzeichnet, dass** das 6,7-disubstituierte 2,2-Dialkylchroman oder -chromen der allgemeinen Formel (I) oder (II) in Mengen von 0,001 - 5 Gew.%, bezogen auf die gesamte Zusammensetzung, eingesetzt wird.

6. Zusammensetzung gemäß einem der vorstehenden Ansprüche, **dadurch gekennzeichnet, dass** die Polyphenole ausgewählt sind aus monomeren Catechinen, Epicatechin und Epigallocatechin.

7. Zusammensetzung gemäß einem der vorstehenden Ansprüche, **dadurch gekennzeichnet, dass** sie konfektioniert ist als ein Leave-on-Produkt wie Creme, Salbe, Pflegestift, Make-up, Lotion, Maske, Packung und Pflasterbeschichtung, Spray, Mousse, die in Form einer O/W-Emulsion, W/O-Emulsion, O/W/O-Emulsion, W/O/W-Emulsion, O/W-Mikroemulsion, W/O-Mikroemulsion, eines wässrigen, alkoholischen oder wässrigalkoholischen Gels sowie als Öl vorliegen kann, oder als ein Rinse-off-Produkt wie Waschgel, Waschcreme, Duschgel, Duschbad, Seife, Shampoo, Haarkonditioniermittel, Haarfärbezusammensetzung, Haarwell- und Haarglättungsmittel sowie Tränkemulsion und -lösung für feste Substrate zur Reinigung und/oder Pflege der Haut und/oder der Haare.

8. Zusammensetzung gemäß einem der vorstehenden Ansprüche, **dadurch gekennzeichnet, dass** die Polyphenole in Mengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt werden.

9. Nicht-therapeutische Verwendung von 6,7-disubstituierten 2,2-Dialkylchromanen oder -chromenen der allgemeinen Formeln (I) oder (II),

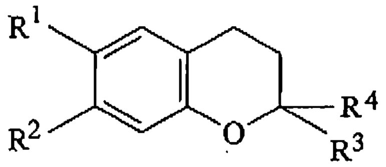

(I)

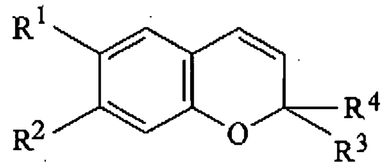

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen,
in topischen kosmetischen und pharmazeutischen Zusammensetzungen zur synergistischen Steigerung der antioxidativen Wirkung von Antioxidantien, ausgewählt aus Polyphenolen.

10. Nicht-therapeutische Verwendung von 6,7-disubstituierten 2,2-Dialkylchromanen oder chromenen der allgemeinen Formeln (I) oder (II),

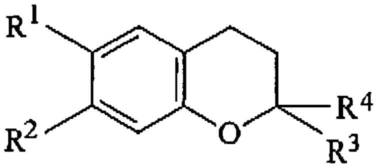

(I)

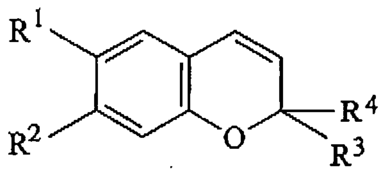

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, in Kombination mit Polyphenolen in topischen kosmetischen und pharmazeutischen Zusammensetzungen zur nicht-therapeutischen Hautpflege und/oder zur nicht-therapeutischen vorbeugenden Behandlung der Hautalterung.

## Claims

1. A cosmetic or pharmaceutical topical composition containing at least one 6,7-disubstituted 2,2-dialkylchromane or 2,2-dialkylchromene of general formula (I) or (II),

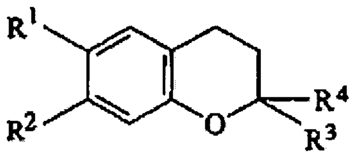

(I)

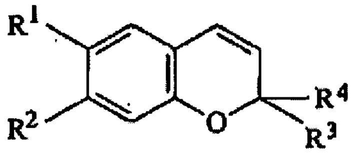

(II)

wherein $R^1$ et $R^2$ independently of each other represent an OH group, a methoxy group or a $CF_3CH_2O$ group and $R^3$ and $R^4$ independently of each other represent a $C_1$-$C_4$ alkyl group and at least one additional antioxidant selected from polyphenols.

2. The composition according to claim 1, **characterised in that** $R^1$ and $R^2$ independently of each other represent an OH group or a methoxy group.

3. The composition according to any of claims 1 or 2, **characterised in that** $R^3$ and $R^4$ independently of each other are selected from a methyl, ethyl, n-propyl, isopropyl, and n-butyl group.

4. The composition according to any of the preceding claims, **characterised in that** $R^1$ represents an OH group, $R^2$ represents a methoxy group and $R^3$ and $R^4$ represent a methyl group.

5. The composition according to any of the preceding claims, **characterised in that** the 6,7-disubstituted 2,2-dialkylchromane or 2,2-dialkylchromene of general formula (I) or (II) is applied in amounts from 0.001 to 5% by weight, based on the total composition.

6. The composition according to any of the preceding claims, **characterised in that** the polyphenols are selected from monomeric catechines, epicatechine and epigallocathechine.

7. The composition according to any of the preceding claims, **characterised in that** it is made as a leave-on product such as a cream, an ointment, a care stick, a make-up, a lotion, a mask, a packaging and a bandage coating, an aerosol, a foam, which may exist as an oil/water emulsion, a water/oil emulsion, an oil/water/oil emulsion, a water/oil/water emulsion, an oil/water micro-emulsion, a water/oil micro-emulsion, an aqueous, alcoholic or aqueous/alcoholic gel, as well as an oil, or as a product to be rinsed such as a washing gel, a washing cream, a shower gel, a shower/bath product, a soap, a shampoo, a hair conditioning agent, a hair dying composition, a hair waving agent, and a hair smoothing agent, an impregnation emulsion and an impregnation solution for hard substrates, for cleaning and/or taking care of the skin and/or hair.

**8.** The composition according to any of the preceding claims, **characterised in that** the polyphenols are applied in amounts from 0.001 to 10% by weight, preferably from 0.005 to 5% by weight, and more preferably from 0.08 to 3%, each based on the total cosmetic composition.

**9.** The non-therapeutic use of 6,7-disubstituted 2,2-dialkylchromanes or 2,2-dialkylchromenes of general formulae (I) or (II),

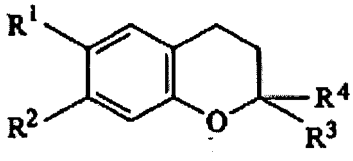

(I)

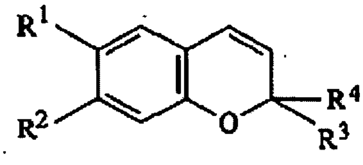

(II)

wherein $R^1$ and $R^2$ independently of each other represent an OH group a methoxy group, or a $CF_3CH_2O$ group, and $R^3$ and $R^4$ independently of each other represent a $C_1$-$C_4$ alkyl group,
in cosmetic and pharmaceutical topical compositions for synergistic increase of the anti-oxidation effect of antioxidants selected from polyphenols.

**10.** The non-therapeutic use of 6,7-disubstituted 2,2-dialkylchromanes or 2,2-dialkylchromenes of general formulae (I) or (II),

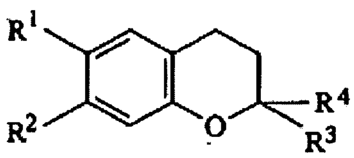

(I)

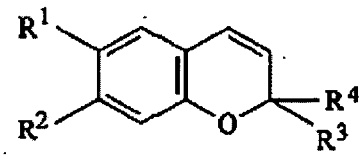

(II)

wherein $R^1$ and $R^2$ independently of each other represent an OH group, a methoxy group, or a $CF_3CH_2O$ group, and $R^3$ and $R^4$ independently of each other represent a $C_1$-$C_4$ alkyl group, in combination with polyphenols in cosmetic and pharmaceutical topical compositions for non-therapeutic care of the skin and/or for non-therapeutic preventive treatment of hair aging.

**Revendications**

**1.** Composition topique cosmétique ou pharmaceutique, contenant au moins un 2,2-dialkylchromane ou 2,2-dialkylchromène disubstitué en 6,7 de formula générale (I) ou (II),

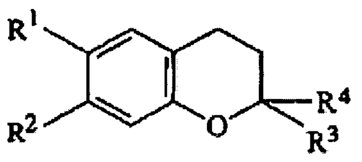

(I)

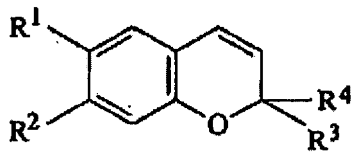

(II)

dans lesquelles $R^1$ et $R^2$ représentent indépendamment l'un de l'autre, un groupe OH, un groupe méthoxy, ou un groupe $CF_3CH_2O$ et $R^3$ et $R^4$ représentent indépendamment l'un de l'autre, un groupe alkyle en $C_1$ à $C_4$, et au moins un antioxydant supplémentaire choisi parmi les polyphénols.

2. Composition selon la revendication 1, **caractérisée en ce que** $R^1$ et $R^2$ représentent indépendamment l'un de l'autre un groupe OH ou un groupe méthoxy.

3. Composition selon l'une quelconque des revendications 1 ou 2, **caractérisée en ce que** $R^3$ et $R^4$ sont choisis indépendamment l'un de l'autre, parmi un groupe méthyle, éthyle, n-propyle, isopropyle, et n-butyle.

4. Composition selon l'une quelconque des revendications précédentes, **caractérisée en ce que** $R^1$ représente un groupe OH, $R^2$ représente un groupe méthoxy et $R^3$ et $R^4$ représentent un groupe méthyle.

5. Composition selon l'une quelconque des revendications précédentes, **caractérisée en ce que** le 2,2-dialkylchromane ou 2,2-dialkylchromène disubstitué en 6,7 de formule générale (I) ou (II) est mis en oeuvre en des quantités de 0,001 à 5% en poids, par rapport à la composition totale.

6. Composition selon l'une quelconque des revendications précédentes, **caractérisée en ce que** les polyphénols sont choisis parmi les catéchines monomères, l'épicatéchine et l'épigallocathéchine.

7. Composition selon l'une quelconque des revendications précédentes, **caractérisée en ce qu'**elle est fabriquée en tant que produit à laisser appliqué comme une crème, une pommade, un bâtonnet de soin, un maquillage, une lotion, un masque, un enveloppement et un revêtement de pansement, un aérosol, une mousse, qui peut exister sous forme d'une émulsion huile/eau, d'une émulsion eau/huile, d'une émulsion huile/eau/huile, d'une émulsion eau/huile/eau, d'une micro-émulsion huile/eau, d'une micro-émulsion eau/huile, d'un gel aqueux, alcoolique ou aqueux/alcoolique, ainsi que sous forme d'huile, ou sous forme de produit à rincer comme un gel de lavage, une crème de lavage, un gel de douche, un produit douche, bain, un savon, un shampoing, un agent de conditionnement capillaire, une composition de teinture capillaire, un agent de permanente capillaire, et un agent de lissage capillaire, une émulsion d'imprégnation et une solution d'imprégnation pour des substrats durs, pour le nettoyage et/ou le soin de la peau et/ou du cheveu.

8. Composition selon l'une quelconque des revendications précédentes, **caractérisée en ce que** les polyphénols sont mis en oeuvre en des quantités de 0,001 à 10% en poids, de préférence de 0,005 à 5% en poids, et de manière particulièrement préférée de 0,08 à 3%, à chaque fois par rapport à la composition cosmétique totale.

9. Utilisation non thérapeutique de 2,2-dialkylchromanes ou 2,2-dialkylchromènes disubstitués en 6,7 de formules générales (I) ou (II),

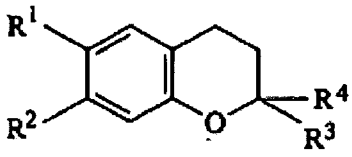

(I)

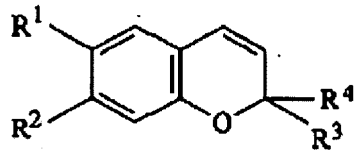

(II)

dans lesquelles $R^1$ et $R^2$ représentent indépendamment l'un de l'autre, un groupe OH, un groupe méthoxy, ou un groupe $CF_3CH_2O$, et $R^3$ et $R^4$ indépendamment l'un de l'autre représentent un groupe alkyle en $C_1$ à $C_4$,
dans des compositions topiques cosmétiques et pharmaceutiques pour l'augmentation synergique de l'effet d'anti-oxydation d'agents antioxydants, choisis parmi les polyphénols.

**10.** Utilisation non thérapeutique de 2,2-dialkylchromanes ou 2,2-dialkylchromènes disubstitués en 6,7 de formules générales (I) ou (II),

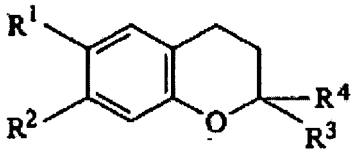

(I)

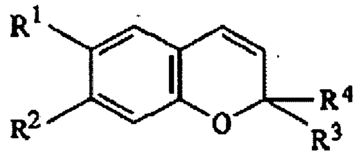

(II)

dans lesquelles $R^1$ et $R^2$ représentent indépendamment l'un de l'autre, un groupe OH, un groupe méthoxy, ou un groupe $CF_3CH_2O$, et $R^3$ et $R^4$ représentent indépendamment l'un de l'autre, un groupe alkyle en $C_1$ à $C_4$, en combinaison avec des polyphénols dans des compositions topiques cosmétiques et pharmaceutiques pour le soin non thérapeutique de la peau et/ou pour le traitement préventif non thérapeutique du vieillissement capillaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1174140 A1
- US 5811083 A
- EP 655239 A1
- EP 1002533 A1
- DE 19756454 A
- DE 4413686 C